# EUROPÄISCHE PATENTSCHRIFT

(11) **EP 3 606 396 B1**
(45) Veröffentlichungstag und Bekanntmachung des Hinweises auf die Patenterteilung: **17.03.2021**
(21) Anmeldenummer: 18717243.2
(22) Anmeldetag: 22.03.2018
(51) Int. Cl.: A61B 1/045, A61B 1/00, H02K 41/02, H01F 7/16, H02K 33/12

(54) **ELEKTROMAGNETISCHER AKTUATOR FÜR EIN CHIRURGISCHES INSTRUMENT UND VERFAHREN ZUM HERSTELLEN DESSELBEN**
ELECTROMAGNETIC ACTUATOR FOR A SURGICAL INSTRUMENT AND METHOD FOR PRODUCING SAME
DISPOSITIF D'ACTIONNEMENT ÉLECTROMAGNÉTIQUE POUR UN INSTRUMENT CHIRURGICAL ET PROCÉDÉ POUR SA FABRICATION

(30) Priorität: 06.04.2017 DE 102017107397
(43) Veröffentlichungstag der Anmeldung: 12.02.2020
(73) Patentinhaber: Olympus Winter & Ibe GmbH, 22045 Hamburg (DE)
(72) Erfinder: WIETERS, Martin, 22885 Barsbüttel (DE)
(74) Vertreter: Seemann & Partner Patentanwälte mbB
(86) Internationale Anmeldenummer: PCT/EP2018/057317
(87) Internationale Veröffentlichungsnummer: WO 2018/184859

(56) Entgegenhaltungen:
- DE-A1-102014 204 736
- US-A- 5 896 076
- US-A1- 2013 314 517

## Beschreibung

Die Erfindung betrifft einen elektromagnetischen Aktuator für ein chirurgisches Instrument, umfassend einen außerhalb eines Rohres angeordneten Stator und einen im Rohr gelagerten Läufer, der in einer Längsaxialrichtung des Rohres in dem Rohr verschiebbar gelagert ist, wobei der Läufer wenigstens teilweise ein paramagnetisches und/oder ferromagnetisches Material umfasst und durch Beaufschlagung mit einem elektromagnetischen Feld in der Längsaxialrichtung verschiebbar ist, wobei der Stator einen distalen Permanentmagneten und einen proximalen Permanentmagneten umfasst, die in Längsaxialrichtung gegensinnig gepolt sind, und zumindest eine elektrische Spule zum Erzeugen des elektromagnetischen Felds umfasst. Ferner betrifft die Erfindung ein chirurgisches Instrument mit einem solchen elektromagnetischen Aktuator. Schließlich betrifft die Erfindung ein Verfahren zum Herstellen eines elektromagnetischen Aktuators für ein chirurgisches Instrument, umfassend einen außerhalb eines Rohres angeordneten Stator und einen im Rohr gelagerten Läufer, der in einer Längsaxialrichtung des Rohres in dem Rohr verschiebbar gelagert ist, wobei der Läufer wenigstens teilweise ein paramagnetisches und/oder ferromagnetisches Material umfasst und durch Beaufschlagung mit einem elektromagnetischen Feld in der Längsaxialrichtung verschiebbar ist, wobei der Stator einen distalen Permanentmagneten und einen proximalen Permanentmagneten umfasst, die in Längsaxialrichtung gegensinnig gepolt sind, und zumindest eine elektrische Spule zum Erzeugen des elektromagnetischen Felds umfasst.

Elektromagnetische Aktuatoren haben vielfältige Anwendungen. Beispielsweise können mit ihnen Schalter betätigt oder Mikrooptiken eingestellt oder verstellt werden. Bei chirurgischen Instrumenten, beispielsweise Endoskopen, können diese kleinbauenden Aktuatoren dazu verwendet werden, um einen Fokus oder eine Vergrößerung eines optischen Systems zu verändern. Bei Endoskopen mit variabler Blickrichtung ist es außerdem möglich mithilfe eines elektromagnetischen Aktuators eine Blickrichtung des optischen Systems einzustellen oder zu verändern. Die Veränderung der optischen Eigenschaften eines optischen Systems erfolgt, indem ein optisches Bauteil, beispielsweise eine Linse, ein Prisma oder eine Blende, durch den Aktuator verschoben wird, wobei sich das optische Bauteil im oder am Läufer des Aktuators befindet.

Es sind bistabile und monostabile elektromagnetische Aktuatoren bekannt. Bei einem bistabilen elektromagnetischen Aktuator ist ein Läufer vorhanden, der in einem Permanentmagnetfeld in einer von zwei extremen Positionen (Endpositionen) gehalten wird und durch Schaltung eines elektromagnetischen Felds aus einer dieser beiden stabilen Position in die jeweils andere stabile Position überführt werden kann. Bei einem monostabilen elektromagnetischen Aktuator wird der Läufer von einem Magnetfeld, welches von einem oder mehreren Permanentmagneten erzeugt wird, stabil in seiner Ruheposition gehalten. Durch Anlegen eines von einer Magnetspule erzeugten elektromagnetischen Felds wird der Läufer aus dieser stabilen Ruheposition heraus bewegt. Bistabile Systeme sind für einen zweistufigen Betrieb mit leistunglos gehaltenen Endpositionen besonders geeignet. Monostabile Systeme sind hingegen für eine kontinuierliche Verstellung gut geeignet.

Ein bistabiler elektromagnetischer Aktuator ist beispielsweise aus der DE 10 2014 204 763 A1 bekannt. Bei diesem elektromagnetischen Aktuatoren sind Spulen, Joche und Magnete nebeneinander im Stator angeordnet, wobei die Spulen und die Joche in einer Längsaxialrichtung zwischen zwei gegensätzlich gepolten Magneten angeordnet sind. Es ist außerdem ein Rückschlusselement vorhanden, welches ebenso wie der Läufer aus einem ferromagnetischen Material hergestellt ist. Der Läufer und der Stator sind durch ein Gleitrohr voneinander getrennt. Dabei sitzt der Stator auf dem Außendurchmesser des Gleitrohres und der Läufer gleitet auf der Innenseite des Gleitrohres in dessen Innendurchmesser.

Aus US 2013/0314517 A1 ist eine Bildaufnahmeeinheit bekannt, bei der eine Linseneinheit mittels eines Schwingspulenmotors bewegt wird.

US 5,896,076 A zeigt einen Aktuator für große Maschinen, bei denen entweder die Magneten am Stator innerhalb der Spule angeordnet sind oder die Spule um den Läufer gewickelt ist.

Diese herkömmlichen Systeme haben in einer Längsaxialrichtung, in der sich der Läufer bewegt, einen relativ großen Stator und infolgedessen auch eine relativ große Baugröße. Dies schränkt die Einsatzmöglichkeiten solcher Antriebe unerwünscht ein.

Es ist daher eine Aufgabe der Erfindung einen elektromagnetischen Aktuator, ein chirurgisches Instrument mit einem elektromagnetischen Aktuator sowie ein Verfahren zum Herstellen eines elektromagnetischen Aktuators anzugeben, wobei der elektromagnetische Aktuator eine kompakte Baugröße aufweisen soll.

Die Aufgabe wird gelöst durch einen elektromagnetischen Aktuator für ein chirurgisches Instrument, umfassend einen außerhalb eines Rohres angeordneten Stator und einen im Rohr gelagerten Läufer, der in einer Längsaxialrichtung des Rohres in dem Rohr verschiebbar gelagert ist, wobei der Läufer wenigstens teilweise ein paramagnetisches und/oder ferromagnetisches Material umfasst und durch Beaufschlagung mit einem elektromagnetischen Feld in der Längsaxialrichtung verschiebbar ist, wobei der Stator einen distalen Permanentmagneten und einen proximalen Permanentmagneten umfasst, die in Längsaxialrichtung gegensinnig gepolt sind, und zumindest eine elektrische Spule zum Erzeugen des elektromagnetischen Felds umfasst, wobei der elektromagnetische Aktuator dadurch fortgebildet ist, dass die Permanentmagneten auf einer dem Rohr abgewandten Außenseite der Spule angeordnet sind.

Durch die Anordnung der Permanentmagnete auf der Außenseite der Spulen ist das System außerdem in Längsaxialrichtung besonders kompakt. Ein solches kompaktes System eignet sich besonders gut für den Einsatz in chirurgischen Instrumenten, wie beispielsweise Endoskopen, ferner beispielsweise Endoskopen mit einem flexiblen, semi-flexiblen oder starren Schaft.

Gemäß einer vorteilhaften Ausführungsform ist der elektromagnetische Aktuator dadurch fortgebildet, dass der distale und der proximale Permanentmagnet ein magnetisches Rückschlusselement für das von der elektrischen Spule erzeugte Magnetfeld bilden. Vorteilhaft erfolgt der magnetische Rückschluss bei einem solchen elektromagnetischen Aktuator über die Permanentmagneten selbst. Hierdurch kann das ansonsten übliche magnetische Rückschlusselement eingespart werden. Der für den elektromagnetischen Aktuator benötigte Bauraum ist somit vorteilhaft geringer.

Gemäß einer weiteren vorteilhaften Ausführungsform ist der elektromagnetische Aktuator dadurch fortgebildet, dass eine dem Rohr abgewandte Außenseite der Permanentmagneten zumindest eine Teilfläche einer dem Rohr abgewandten Außenseite des Stators bildet. Mit anderen Worten umfasst der elektromagnetische Aktuator gemäß dieser Ausführungsform kein magnetisches Rückschlusselement, welches traditionell auf der dem Rohr abgewandten Außenseite des Aktuators angeordnet ist. Diese Konstruktion ermöglicht eine besonders kompakte Bauform des elektromagnetischen Aktuators, insbesondere einen besonders kleinen Durchmesser des Systems.

Vorteilhaft ist ferner vorgesehen, dass ein distales Ende des Stators von einem distalen Statorpolschuh und ein in Längsaxialrichtung gegenüberliegendes proximales Ende von einem proximalen Statorpolschuh gebildet sind. Durch die Verwendung von Statorpolschuhen wird der Wirkungsgrad des elektromagnetischen Aktuators erhöht. In der Folge können vorteilhaft größere Haltekräfte bereitgestellt oder geringere Stellströme eingesetzt werden.

In einer vorteilhaften Ausführungsform zeichnet sich der elektromagnetische Aktuator dadurch aus, dass der Stator einen zentralen Statorpolschuh umfasst, der in Längsaxialrichtung zwischen den Permanentmagneten angeordnet ist. Durch den Einsatz des zentralen Statorpolschuhs wird der Wirkungsgrad des magnetischen Systems weiter verbessert. Insbesondere für ein bistabiles System ist die Verwendung eines zentralen Statorpolschuhs vorteilhaft. In dem zentralen Statorpolschuh werden die von den gegensätzlich gepolten Permanentmagneten erzeugten magnetischen Flüsse aus dem Bereich des Läufers zurück in Richtung der Permanentmagneten geführt.

Insbesondere ist der zentrale Statorpolschuh dicker als der oder die äußeren Statorpolschuhe, d. h. der distale Statorpolschuh oder der proximale Statorpolschuh. Beispielsweise weist der zentrale Statorpolschuh eine Materialstärke, gemessen in Längsaxialrichtung, auf, welche 1,2-fach bis doppelt so groß ist wie die in gleicher Richtung gemessene Materialstärke der äußeren Polschuhe. Es ist ferner insbesondere vorgesehen, dass der distale Statorpolschuh und der proximale Statorpolschuh die gleiche Materialstärke, gemessen erneut in Längsaxialrichtung, aufweisen.

Gemäß einer weiteren vorteilhaften Ausführungsform ist der elektromagnetische Aktuator dadurch fortgebildet, dass sich der zentrale Statorpolschuh in einer zu der Längsaxialrichtung senkrechten Radialrichtung von der dem Rohr zugewandten Innenseite der Spule bis zu der dem Rohr abgewandten Außenseite der Permanentmagneten erstreckt und die Spule eine distale Spule und eine proximale Spule umfasst, wobei sich die beiden Spulen in Längsaxialrichtung zu beiden Seiten des zentralen Statorpolschuhs erstrecken und elektrisch so miteinander verbunden sind, dass die distale Spule ein erstes Magnetfeld erzeugt, welches gleich zu einem von der proximalen Spule erzeugten zweiten Magnetfeld orientiert ist.

Das erste Magnetfeld und das zweite Magnetfeld sind mit anderen Worten gleichsinnig orientiert. Die Dimensionierung der Polschuhe erlaubt eine besonders effiziente Flussführung und erhöht dadurch den Wirkungsgrad des elektromagnetischen Aktuators weiter.

Ferner ist vorteilhaft und insbesondere vorgesehen, dass der zentrale Statorpolschuh aus einem proximalen zentralen Stator-Teil-Polschuh und aus einem distalen zentralen Stator-Teil-Polschuh gebildet ist. Eine solche Konstruktion erlaubt vorteilhaft die Bildung einer distalen Baugruppe und einer proximalen Baugruppe. Die Verwendung von Baugruppen vereinfacht und beschleunigt die Herstellung des elektromagnetischen Aktuators.

Gemäß einer weiteren vorteilhaften Ausführungsform ist zwischen dem distalen zentralen Stator-Teil-Polschuh und dem proximalen zentralen Stator-Teil-Polschuh ein Luftspalt vorhanden. Die distale und die proximale Baugruppe sind mit anderen Worten mechanisch nicht miteinander verbunden. Da die Permanentmagneten der beiden Baugruppen sich gegenseitig abstoßen, richten sich die beiden Baugruppen selbstständig und unabhängig von gegebenenfalls vorhandenen Bauteiltoleranzen an einem distalen und an einem proximalen Anschlag aus.

Gemäß einer alternativen Ausführungsform und für den Fall, dass eine mechanische Verbindung der beiden Baugruppen gewünscht ist, ist zwischen den Baugruppen ein Klebstoff vorhanden, der keine oder eine besonders geringe Volumenschrumpfung während des Aushärtens zeigt. Geeignet ist beispielsweise ein Klebstoff, der während des Aushärtens weniger als 5 % Volumen einbüßt.

Gemäß einer vorteilhaften Ausführungsform ist der elektromagnetische Aktuator dadurch fortgebildet, dass der distale Statorpolschuh, die distale Spule, der distale Permanentmagnet und der distale zentrale Stator-Teil-Polschuh eine vorgefertigte distale Baugruppe bilden und der proximale zentrale Stator-Teil-Polschuh, die proximale Spule, der proximale Permanentmagnet und der proximale Statorpolschuh eine vorgefertigte proximale Baugruppe bilden, wobei insbesondere die Bauteile der distalen und/oder der proximalen Baugruppe miteinander verklebt sind.

Es ist ferner insbesondere vorgesehen, dass die distale Baugruppe und die proximale Baugruppe identisch zueinander aufgebaut sind. Um die gegensätzliche magnetische Orientierung der Permanentmagneten in dem elektromagnetischen Aktuator zu realisieren, ist beim Zusammenbau vorgesehen, dass entweder die proximale Baugruppe oder die distale Baugruppe gegenüber der jeweils anderen Baugruppe um 180° gedreht eingebaut wird. Ferner ist in diesem Zusammenhang zu beachten, dass die in den Baugruppen vorhandenen Magnetspulen passend verdrahtet werden, so dass diese gleich orientierte Magnetfelder erzeugen.

Die Verwendung von Baugruppen beschleunigt die Herstellung des elektromagnetischen Aktuators. Besonders vorteilhaft ist es, wenn gemäß einer weiteren Ausführungsform die Baugruppen vorgefertigte Bauelemente sind.

Gemäß einer weiteren vorteilhaften Ausführungsform ist vorgesehen, dass sich zumindest einer der Statorpolschuhe in einer zu der Längsaxialrichtung senkrechten Radialrichtung von einer dem Rohr zugewandten Innenseite der Spule bis zu einer dem Rohr abgewandten Außenseite der Permanentmagneten erstreckt. Insbesondere ist vorgesehen, dass sich alle Statorpolschuhe in Radialrichtung von der Außenseite des Rohrs, welche an der Innenseite der Spule anliegt und daher die gleiche Dimension definiert, bis zu einer dem Rohr abgewandten Außenseite der Permanentmagneten erstrecken. Diese Dimensionierung der Statorpolschuhe erlaubt eine besonders effiziente Flussführung der magnetischen Felder.

Insbesondere ist ferner vorgesehen, dass die Permanentmagneten Ringmagnete sind, die die Spulen umschließen. Gemäß einer alternativen Ausführungsform ist vorgesehen, dass der distale und der proximale Permanentmagnet jeweils aus zumindest einem Stabmagnet gebildet sind.

Die Verwendung von Stabmagneten ist besonders vorteilhaft, da der von dem elektromagnetischen Aktuator beanspruchte Bauraum auf diese Weise verringert werden kann. Läufer und Spule können querschnittskonstant ausgeführt werden und die darüber, also radial weiter außen, angeordneten Magnete benötigen keinen umlaufenden Bauraum mehr. So ist es möglich, dass in dem freiwerdenden Bauraum, insbesondere wenn der elektromagnetische Aktuator in einem Endoskopschaft eingesetzt wird, weitere Bauelemente des Endoskops untergebracht werden. Ferner sind Stabmagneten mechanisch deutlich stabiler als Magnetringe, die bauartbedingt eine sehr geringe Wandstärke aufweisen müssen. Die Handhabung der Stabmagnete ist also wesentlich einfacher als die von Magnetringen, was die Herstellung des elektromagnetischen Aktuators vereinfacht und beschleunigt.

Ferner ist insbesondere vorgesehen, dass der distale und der proximale Permanentmagnet jeweils aus mehreren Stabmagneten gebildet ist, wobei die den distalen und/oder den proximalen Permanentmagneten bildenden Stabmagneten gleichmäßig beabstandet entlang des Umfangs des Rohrs angeordnet sind. Durch die Verwendung einer Vielzahl von Stabmagneten kann die bereitgestellte magnetische Kraft erhöht werden, da durch mehrere Magnete ein größerer magnetischer Fluss erzeugbar ist. Durch die gleichmäßige Beabstandung der Stabmagnete kann ein besonders homogenes Magnetfeld erzeugt werden

Gemäß einer weiteren vorteilhaften Ausführungsform ist der elektromagnetische Aktuator dadurch fortgebildet, dass zumindest einer der Permanentmagnete hartmagnetische Partikel umfasst, die in einer Kunststoffmatrix eingebettet sind, wobei dieser Permanentmagnet insbesondere in einem Spritzgussverfahren hergestellt ist. Als magnetische Partikel sind beispielsweise NdFeB-Partikel (Neodym, Eisen, Bohr) oder eine Mischung aus Partikeln dieser Materialien geeignet, welche beispielsweise in einen Epoxidharzklebstoff eingerührt werden. Zur Herstellung der Permanentmagnete wird beispielsweise ein Freiraum zwischen den Statorpolschuhen ausgegossen, der von den Permanentmagneten eingenommen werden soll. Dies ist besonders vorteilhaft, wenn der elektromagnetische Aktuator eine vorgefertigte distale und proximale Baugruppe umfasst. Während der Herstellung der Baugruppen kann auf diese Weise nicht nur das magnetische Material eingebracht werden, sondern es wird im selben Schritt auch die Fixierung der Statorpolschuhe und der Spule vorbereitet bzw. vorgenommen. Ferner ist insbesondere vorgesehen, dass die gesamte Baugruppe anschließend aufmagnetisiert wird, so dass die magnetischen Partikel eine gewünschte magnetische Orientierung einnehmen.

Gemäß einer vorteilhaften Weiterbildung ist insbesondere vorgesehen, dass in zumindest einem Permanentmagneten zumindest ein Spulendraht der Spule eingegossen ist. Mit anderen Worten werden die Zuleitungen der Spule durch den Permanentmagneten geführt. So kann vorteilhaft der ansonsten für die Zuleitungen der Magnetspulen erforderliche Bauraum eingespart werden.

Ferner ist insbesondere vorgesehen, dass der Läufer in Längsaxialrichtung eine Gesamtlänge aufweist, die kleiner ist als eine maximale Ausdehnung des Stators in dieser Richtung. Durch eine solche Dimensionierung des Stators und des Läufers kann ein bistabiler elektromagnetischer Aktuator angegeben werden. Es ist ebenso möglich, andere Auslegungen zu finden, in denen der elektromagnetische Aktuator bistabil ist. In diesem Zusammenhang sind beispielsweise die Auslegung und Größe der Permanentmagnete und der Spulen von Bedeutung. Gemäß einer weiteren Ausführungsform ist vorgesehen, dass der Läufer in Längsaxialrichtung eine größere Ausdehnung als der Stator aufweist. Durch eine solche Auslegung des elektromagnetischen Aktuators kann ein monostabiler Aktuators realisiert werden.

Die Aufgabe wird ferner gelöst durch ein chirurgisches Instrument, insbesondere ein Endoskop, mit einem elektromagnetischen Aktuator nach einem oder mehreren der zuvor genannten Ausführungsformen.

Auf das chirurgische Instrument treffen gleiche oder ähnliche Vorteile zu, wie sie bereits im Hinblick auf den elektromagnetischen Aktuator erwähnt wurden, so dass auf Wiederholungen verzichtet werden soll.

Außerdem wird die Aufgabe gelöst durch ein Verfahren zum Herstellen eines elektromagnetischen Aktuators für ein chirurgisches Instrument, umfassend einen außerhalb eines Rohres angeordneten Stator und einen im Rohr gelagerten Läufer, der in einer Längsaxialrichtung des Rohres in dem Rohr verschiebbar gelagert ist, wobei der Läufer wenigstens teilweise ein paramagnetisches und/oder ferromagnetisches Material umfasst und durch Beaufschlagung mit einem elektromagnetischen Feld in der Längsaxialrichtung verschiebbar ist, wobei der Stator einen distalen Permanentmagneten und einen proximalen Permanentmagneten umfasst, die in Längsaxialrichtung gegensinnig gepolt sind, und zumindest eine elektrische Spule zum Erzeugen des elektromagnetischen Felds umfasst, wobei das Verfahren dadurch fortgebildet ist, dass die Permanentmagneten auf einer dem Rohr abgewandten Außenseite der Spule angeordnet werden.

Auch auf das Verfahren zum Herstellen eines elektromagnetischen Aktuators treffen gleiche oder ähnliche Vorteile zu wie sie bereits im Hinblick auf den elektromagnetischen Aktuator erwähnt wurden. Gemäß einer vorteilhaften Ausführungsform ist vorgesehen, dass die Permanentmagnete derart angeordnet werden, so dass eine dem Rohr abgewandte Außenseite der Permanentmagneten zumindest eine Teilfläche einer dem Rohr abgewandten Außenseite des Stators bilden. Mit anderen Worten wird während des Verfahrens zum Herstellen des elektromagnetischen Aktuators kein magnetisches Rückschlusselement, insbesondere kein magnetisches Rückschlusselement, welches auf der Außenseite des Stators vorhanden ist, vorgesehen.

Ferner ist insbesondere vorgesehen, dass ein distaler Statorpolschuh distal des distalen Permanentmagneten angeordnet wird und ein distales Ende des Stators bildet und ein proximaler Statorpolschuh proximal des proximalen Permanentmagneten angeordnet wird und ein in Längsaxialrichtung gegenüberliegendes proximales Ende bildet. Vorteilhaft ist ferner vorgesehen, dass ein zentraler Statorpolschuh in Längsaxialrichtung zwischen den Permanentmagneten angeordnet wird.

Gemäß einer weiteren vorteilhaften Ausführungsform ist das Verfahren dadurch fortgebildet, dass sich der zentrale Statorpolschuh in einer zu der Längsaxialrichtung senkrechten Radialrichtung von der dem Rohr zugewandten Innenseite der Spule bis zu der dem Rohr abgewandten Außenseite der Permanentmagneten erstreckt und die Spule eine distale Spule und eine proximale Spule umfasst, wobei der distale Statorpolschuh, die distale Spule, der distale Permanentmagnet und der distale zentrale Stator-Teil-Polschuh zu einer vorgefertigten distalen Baugruppe und der proximale zentrale Stator-Teil-Polschuh, die proximale Spule, der proximale Permanentmagnet und der proximale Statorpolschuh zu einer vorgefertigten proximalen Baugruppe zusammengefügt werden, wobei insbesondere die Bauteile der distalen und/oder der proximalen Baugruppe miteinander verklebt werden, und anschließend die vorgefertigt proximale Baugruppe und die vorgefertigte distale Baugruppe montiert werden, wobei die beiden Spulen elektrisch so miteinander verbunden werden, dass die distale Spule ein erstes Magnetfeld erzeugt, welches gleich zu einem von der proximalen Spule erzeugten zweiten Magnetfeld orientiert ist und der zentrale Statorpolschuh aus dem proximalen zentralen Stator-Teil-Polschuh und aus dem distalen zentralen Stator-Teil-Polschuh gebildet ist.

Die Verwendung zweier Baugruppen ermöglicht eine schnellere und effizientere Montage. Gleichzeitig wird die Ausrichtung/Justage des proximalen Statorpolschuhs zum proximalen Anschlag und auch die Ausrichtung/Justage des distalen Statorpolschuhs zum distalen Anschlag einfacher und wesentlich genauer. Die Ausrichtung der Statorpolschuhe zu den Anschlägen ist für die korrekte Funktion des elektromagnetischen Aktuators besonders wichtig. Idealerweise liegt die distale Seite des Statorpolschuhs an der distalen Kante des Läufers, also am distalen Anschlag. Das gleiche gilt für die Ausrichtung des proximalen Statorpolschuhs und des proximalen Anschlags. Herkömmlich werden die distalen und proximalen Statorpolschuhe unabhängig voneinander zu den Anschlägen des Läufers ausgerichtet. Der proximale Polschuh wird zum proximalen Anschlag ausgerichtet. Der distale Statorpolschuh wird über die Bauteile des Stators, soweit im Rahmen der Bauteiltoleranzen möglich, ausgerichtet. Bei diesem Vorgang summieren sich die Fertigungstoleranzen der Bauteile auf. Dies wirkt sich entsprechend negativ auf die Positionierung des distalen Bauteils aus. Gleiches gilt selbstverständlich auch für die Positionierung des proximalen Bauteils.

Bei der Verwendung zweier unabhängiger Baugruppen, wobei die Baugruppen einander entgegengesetzt gepolte Permanentmagnete aufweisen, kann die Ausrichtung des proximalen und des distalen Statorpolschuhs unabhängig voneinander zum Gleitrohr bzw. zu den Anschlägen erfolgen. Die vor der Endmontage als fertige Baugruppen hergestellten Einheiten werden entgegengesetzt zueinander eingebaut, so dass sich die Permanentmagneten gegenseitig abstoßen. Beim Zusammenschieben der beiden Baugruppen wirkt auf die Baugruppen eine abstoßende Kraft. Dies hat zur Folge, dass sich die distale Baugruppe in Richtung eines distalen Anschlags und die proximale Baugruppe in Richtung eines proximalen Anschlags solange verschiebt, bis der entsprechende Anschlag erreicht ist. Hierdurch richten sich die beiden Baugruppen in der gewünschten Position aus.

Gemäß einer weiteren vorteilhaften Ausführungsform erfolgt die Montage derart, dass zwischen den Baugruppen, also zwischen dem proximalen zentralen Stator-Teil-Polschuh der distalen Baugruppe und dem distalen zentralen Stator-Teil-Polschuh der proximalen Baugruppe ein Luftspalt vorgesehen wird. Die distale und die proximale Baugruppe sind also nicht miteinander verklebt oder anderweitig mechanisch verbunden. Klebstoff hat stets die Eigenschaft, dass beim Aushärten sein Volumen schrumpft, er also im ausgehärteten Zustand ein geringeres Volumen einnimmt als im nicht ausgehärteten Zustand. Dieser Schrumpfungseffekt würde die einmal erreichte Ausrichtung der beiden Baugruppen zueinander erneut verschlechtern. Somit ist der Verzicht auf einen Klebstoff zwischen den beiden Baugruppen besonders vorteilhaft.

Es ist ferner insbesondere vorgesehen, dass zur Verbindung der beiden Baugruppen ein Klebstoff zum Einsatz kommt, der eine besonders geringe Volumenschrumpfung während des Aushärtvorgangs zeigt. Beispielsweise sind Klebstoffe bekannt, die weniger als 5 % ihres Volumens während des Aushärtens verlieren. Ein solcher Klebstoff ist beispielsweise zur Verbindung der beiden Baugruppen geeignet.

Gemäß einer weiteren vorteilhaften Ausführungsform ist vorgesehen, dass die erste und/oder die zweite Baugruppe mit einem Permanentmagneten hergestellt werden/wird, der hartmagnetische Partikel umfasst, die in einer Kunststoffmatrix eingebettet sind, wobei dieser Permanentmagnet in einem Spritzgussverfahren hergestellt wird und die Kunststoffmatrix gleichzeitig als Klebstoff für die Bauteile der ersten und/oder zweiten Baugruppe dient. Vorteilhaft werden in einem Arbeitsschritt sowohl der Permanentmagnet hergestellt als auch die Bauteile der Baugruppen miteinander verbunden.

Die Verwendung von hartmagnetischen Partikeln, die in einer Kunststoffmatrix eingebettet sind, zur Herstellung der Permanentmagneten ist ferner vorteilhaft, da in zumindest einem Permanentmagneten zumindest ein Spulendraht der Spule eingegossen wird. So kann Bauraum zur Durchführung der Anschlussleitungen der Spulen eingespart werden.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: ein Endoskop als beispielhaftes chirurgisches Instrument in einer schematisch vereinfachten Perspektivansicht,
- Fig. 2: einen elektromagnetischen Aktuator in einem unbestromten Zustand, dessen Läufer sich in einer Endposition befindet, in einer schematisch vereinfachten Längsschnittansicht,
- Fig. 3: den elektromagnetischen Aktuator in bestromtem Zustand, wobei sich dessen Läufer in der entgegengesetzten Endposition befindet, ebenfalls in einer schematisch vereinfachten Längsschnittansicht,
- Fig. 4: einen weiteren elektromagnetischen Aktuator in perspektivischer schematisch vereinfachter Darstellung,
- Fig. 5: den elektromagnetischen Aktuator in einer schematisch vereinfachten geschnittenen perspektivischen Ansicht, welche die Ansicht auf den Innenraum des Aktuators freigibt,
- Fig. 6: einen weiteren elektromagnetischen Aktuator, aufgebaut aus einer proximalen und einer distalen Baugruppe im eingebauten Zustand in einem Endoskoprohr, in einer schematisch vereinfachten Längsschnittansicht durch das Endoskoprohr,
- Fig. 7: diesen in das Endoskoprohr eingebauten elektromagnetischen Aktuator in einer schematisch vereinfachten perspektivischen Schnittansicht,
- Fig. 8: eine vorgefertigte Baugruppe des elektromagnetischen Aktuators in einer schematisch vereinfachten perspektivischen Darstellung, und
- Fig. 9: diese Baugruppe in geschnittener schematisch vereinfachter perspektivischer Darstellung.

In den Zeichnungen sind jeweils gleiche oder gleichartige Elemente und/oder Teile mit denselben Bezugsziffern versehen, so dass von einer erneuten Vorstellung jeweils abgesehen wird.

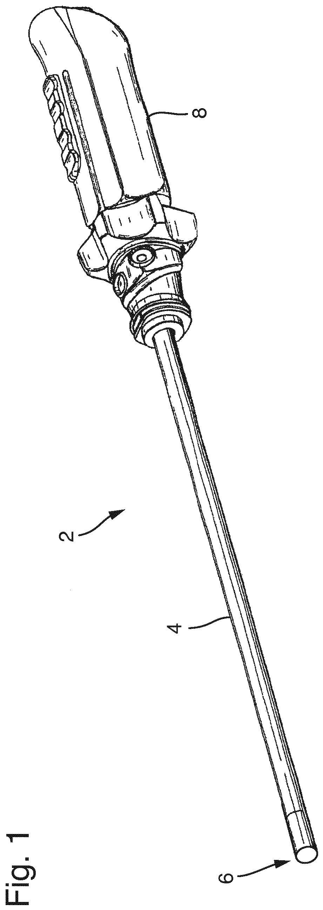

Figur 1 zeigt in einer schematisch vereinfachten Perspektivansicht ein Endoskop 2 als beispielhaftes chirurgisches Instrument. Das Endoskop 2 umfasst einen Endoskopschaft 4, in dem ein optisches System angeordnet ist, mit dem ein vor einem distalen Ende 6 des Endoskopschafts 4 liegendes Operations- oder Beobachtungsfeld beispielsweise auf einen Bildsensor abgebildet wird. An einem proximalen Ende des Endoskops 2 befindet sich ein Handgriff 8. Das in dem Endoskopschaft 4 angeordnete optische System, welches in Figur 1 nicht dargestellt ist, umfasst einen elektromagnetischen Aktuator.

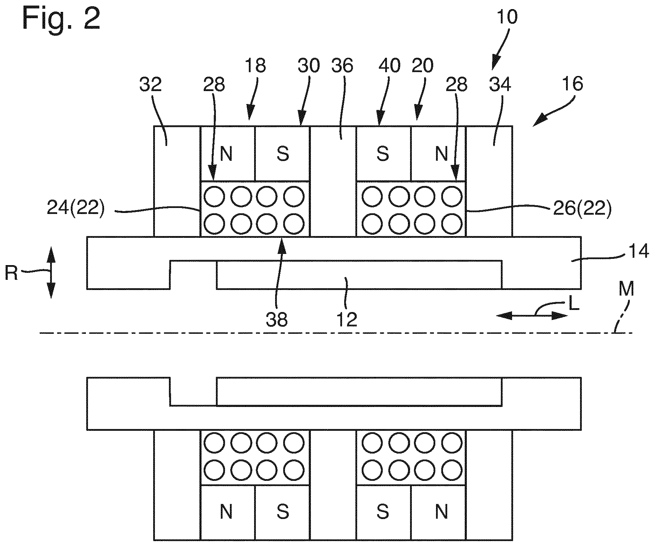

Figur 2 zeigt einen solchen elektromagnetischen Aktuator 10 in einer schematisch vereinfachten Längsschnittansicht. Der elektromagnetische Aktuator 10 ist in einem unbestromten Zustand gezeigt, in dem sich dessen Läufer 12 in einer proximalen Endposition befindet. Der Läufer 12 ist innerhalb eines Rohrs 14 in einer Längsaxialrichtung L verschiebbar aufgenommen. Außerhalb des Rohrs 14 ist ein Stator 16 des elektromagnetischen Aktuators 10 angeordnet. Der Läufer 12 umfasst wenigstens teilweise ein paramagnetisches und/oder ferromagnetisches Material. Bei Beaufschlagung des Läufers 12 mit einem elektromagnetischen Feld ist dieser in der Längsaxialrichtung L innerhalb des Rohrs 14 verschiebbar.

Der Stator 16 umfasst einen distalen Permanentmagneten 18 und einen proximalen Permanentmagneten 20. Beispielhaft handelt es sich bei diesen Magneten 18, 20 um Ringmagneten. Die Magneten 18, 20 sind in Längsaxialrichtung L gepolt, d. h. ihre Nord-Süd-Richtung liegt zumindest näherungsweise parallel zu der Längsaxialrichtung L. Der distale Permanentmagnet 18 und der proximale Permanentmagnet 20 sind so in dem elektromagnetischen Aktuator 10 angeordnet, dass diese gegensinnig gepolt sind. Ferner ist zumindest eine elektrische Spule 22 umfasst, die zum Erzeugen eines elektromagnetischen Felds dient und im dargestellten Ausführungsbeispiel aus einer distalen Spule 24 und einer proximalen Spule 26 aufgebaut ist. Die Permanentmagneten 18, 20 sind auf einer dem Rohr 14 abgewandten Außenseite 28 der Spule 22 angeordnet.

Durch diese Anordnung dient der distale Permanentmagnet 18 und der proximale Permanentmagnet 20 als magnetisches Rückschlusselement für das von der elektrischen Spule 22 erzeugte Magnetfeld. Die Permanentmagneten 18, 20 bilden zumindest eine Teilfläche einer dem Rohr 14 abgewandten Außenseite 30 des Stators 16.

Der in Figur 2 gezeigte elektromagnetische Aktuator ist bezüglich seiner Mittenlängsachse M (strichpunktiert dargestellt) rotationssymmetrisch aufgebaut. Die genannten Bezugszeichen sind daher ausschließlich im oberen Teil der Darstellung aufgeführt, gelten aber ebenso für die entsprechenden Bauteile in der unteren Hälfte.

Der Stator 16 umfasst an seinem distalen Ende einen distalen Statorpolschuh 32 und an seinem in Längsaxialrichtung L gegenüberliegenden proximalen Ende einen proximalen Statorpolschuh 34. Ferner umfasst der Stator 16 einen zentralen Statorpolschuh 36, der in Längsaxialrichtung L zwischen den Permanentmagneten 18, 20 angeordnet ist.

Der zentrale Statorpolschuh 36, der distale Statorpolschuh 32 und der proximale Statorpolschuh 34 erstrecken sich in einer zu der Längsaxialrichtung L senkrechten Radialrichtung R von einer dem Rohr 14 zugewandten Innenseite 38 der Spule 22 bis zu einer dem Rohr 14 abgewandten Außenseite 40 der Permanentmagneten 18, 20.

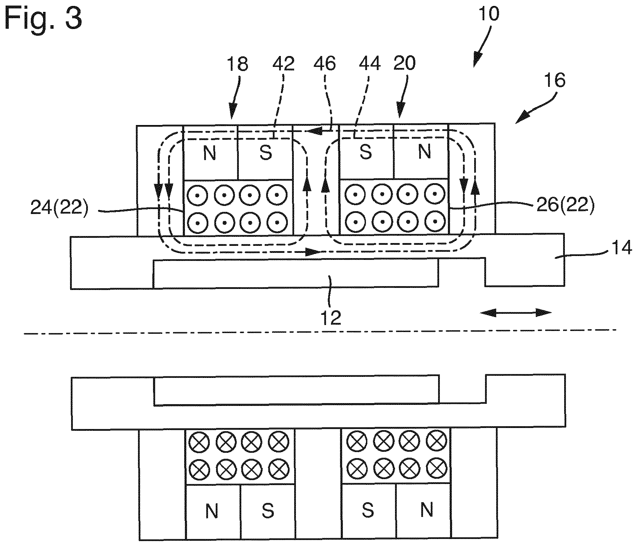

Figur 3 zeigt den aus Figur 2 bekannten elektromagnetischen Aktuator 10 ebenfalls in einer schematisch vereinfachten Längsschnittansicht. Die Spule 22, d. h. die distale Spule 24 und die proximale Spule 26, sind in dem dargestellten Ausführungsbeispiel bestromt. Die distale Spule 24 und die proximale Spule 26 sind elektrisch so gekoppelt, dass ein von der jeweiligen Spule 24, 26 erzeugtes erstes Magnetfeld und zweites Magnetfeld gleich orientiert sind. Dies ergibt sich aus der gleichen Bestromung der distalen Spule 24 und der proximalen Spule 26. Die Stromflussrichtung ist in den schematisch skizzierten Leitern der Spulen 24, 26 angedeutet. Eine aus der Zeichenebene herauszeigende Stromrichtung ist durch einen Punkt und eine in die Zeichenebene hineingerichtete Stromrichtung durch ein Kreuz angedeutet. Das erste Magnetfeld und das zweite Magnetfeld addieren sich zu einem elektromagnetischen Feld 46 der Spule 22.

Das elektromagnetische Feld 46 der Spule 22 überlagert ein erstes statisches Magnetfeld 42 des distalen Permanentmagneten 18 und ein zweites statisches Magnetfeld 44 des proximalen Permanentmagneten 20. Am distalen Ende des Stators 16 überlagern sich das elektromagnetische Feld 46 der Spule 22 und das erste statische Magnetfeld 42 des distalen Permanentmagneten 18 konstruktiv, während sich am gegenüberliegenden proximalen Ende des Stators 16 das Magnetfeld 46 der Spule 22 und das zweite statische Magnetfeld 44 des proximalen Permanentmagneten 20 destruktiv überlagern bzw. abschwächen. Befindet sich der Läufer 12 in der in Figur 2 dargestellten Position, so wirkt in dem Spalt zwischen dem Läufer 12 und dem Rohr 14 eine Kraft, die den Läufer 12 in die in Figur 3 gezeigte Position verlagert.

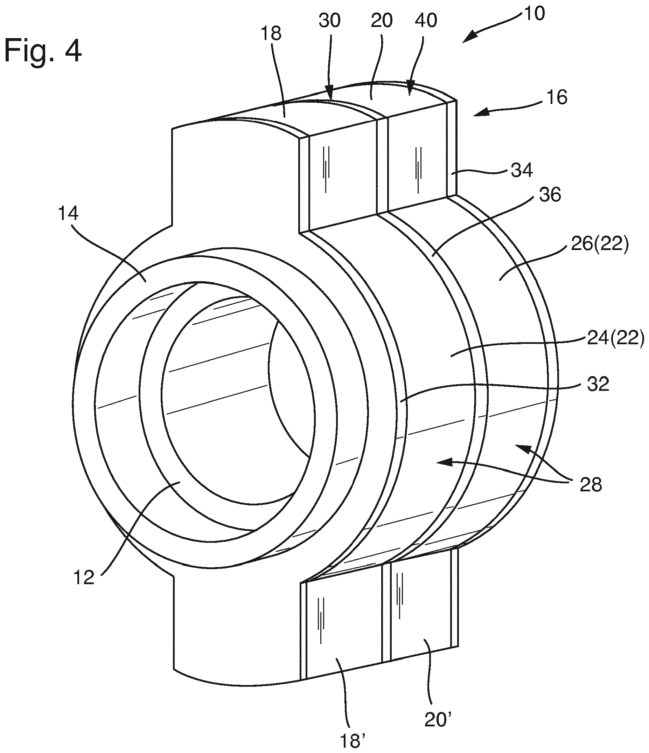

Figur 4 zeigt einen weiteren elektromagnetischen Aktuator 10 in einer schematisch vereinfacht perspektivischen Darstellung. Der distale Permanentmagnet 18 und der proximale Permanentmagnet 20 sind im dargestellten Ausführungsbeispiel als Stabmagnete oder Magnetblöcke ausgeführt. Dabei sind sowohl der distale Permanentmagnet 18 als auch der proximale Permanentmagnet 20 aus mehreren Einzelmagneten aufgebaut. Beispielsweise umfasst der distale Permanentmagnet die mit Bezugszeichen 18 und 18' bezeichneten Bauteile und der proximale Permanentmagnet die mit 20 und 20' bezeichneten Bauteile. Diese Bauteile sind beispielsweise gleichmäßig beabstandet entlang des Umfangs des Rohrs 14 angeordnet. Alternativ zu den gezeigten Stabmagneten können auch ringförmige Permanentmagnete vorgesehen sein, welche sich vollständig entlang des Umfangs an der Außenseite 28 der Spulen 22 erstrecken.

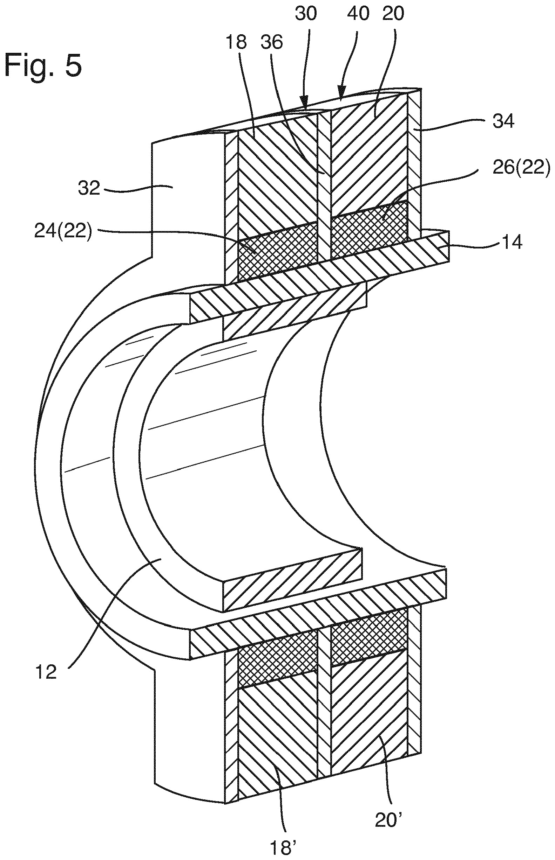

Figur 5 zeigt den aus Figur 4 bekannten elektromagnetischen Aktuator 10 in einer schematisch vereinfachten Schnittansicht, welche den Blick auf den Innenraum des Aktuators freigibt. In dem Läufer 12 ist beispielsweise ein optisches Element, wie etwa eine beispielhaft dargestellte Linse 62 oder ein Prisma, eines optischen Systems aufgenommen. Mithilfe des elektromagnetischen Aktuators 10 kann beispielsweise der Fokus eines optischen Systems verändert werden. Ebenso ist denkbar, dass die Linse oder Linsengruppe eines solchen optischen Systems mithilfe des elektromagnetischen Aktuators 10 verstellt werden, beispielsweise um ein Vario-Objektiv bereitzustellen.

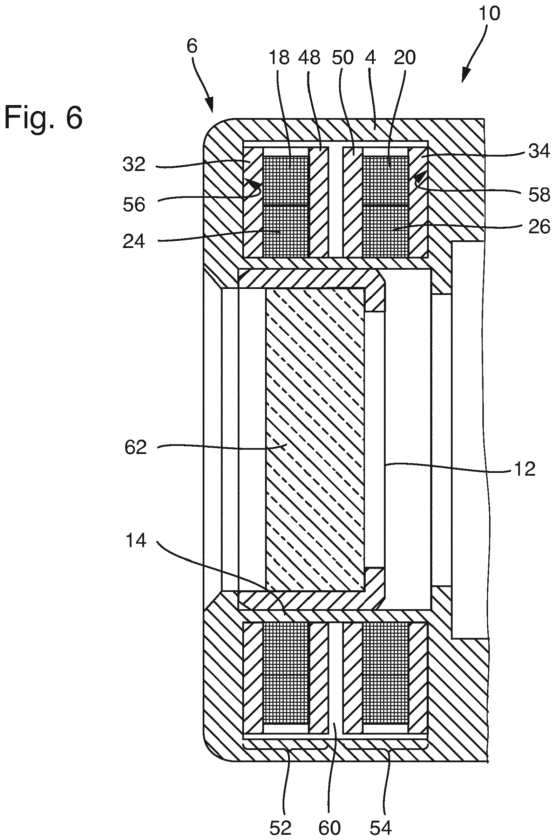

Der zentrale Statorpolschuh 36 ist gemäß weiterer Ausführungsbeispiele aus einem proximalen zentralen Stator-Teil-Polschuh 50 und einem distalen zentralen Stator-Teil-Polschuh 48 gebildet. Figur 6 zeigt einen entsprechenden elektromagnetischen Aktuator 10, bei dem dies realisiert ist. Der elektromagnetische Aktuator 10 ist in einem eingebauten Zustand dargestellt, er befindet sich in einem Endoskopschaft 4 an einem distalen Ende 6 desselben.

Der elektromagnetische Aktuator 10 umfasst eine proximale Baugruppe 54 und eine distale Baugruppe 52. Der distale Statorpolschuh 32, die distale Spule 24, der distale Permanentmagnet 18 und der distale zentrale Stator-Teil-Polschuh 48 bilden die distale Baugruppe 52. Der proximale zentrale Stator-Teil-Polschuh 50, die proximale Spule 26, der proximale Permanentmagnet 20 und der proximale Statorpolschuh 34 bilden die proximale Baugruppe 54. Bei den Baugruppen 52, 54 handelt es sich beispielsweise um vorgefertigte Bauteile. Diese werden mit anderen Worten zusammengebaut, bevor sie im Endoskopschaft 4 zu der gezeigten Anordnung zusammengesetzt werden. Die Bauteile der distalen und/oder der proximalen Baugruppe 52, 54 sind ferner beispielsweise miteinander verklebt.

Da die Permanentmagnete 18, 20 einander entgegengesetzt gepolt sind, stoßen sich die erste und die zweite Baugruppe 52, 54 ab. Die abstoßenden Kräfte drücken die distale Baugruppe 52 an einen distalen Anschlag 56, der im dargestellten Ausführungsbeispiel ein Kragen des Endoskopschafts 4 ist. Die proximale Baugruppe 54 wird gegen einen proximalen Anschlag 58 gedrückt. Zwischen dem distalen zentralen Stator-Teil-Polschuh 48 und dem proximalen zentralen Stator-Teil-Polschuh 50 verbleibt beispielsweise ein Luftspalt 60.

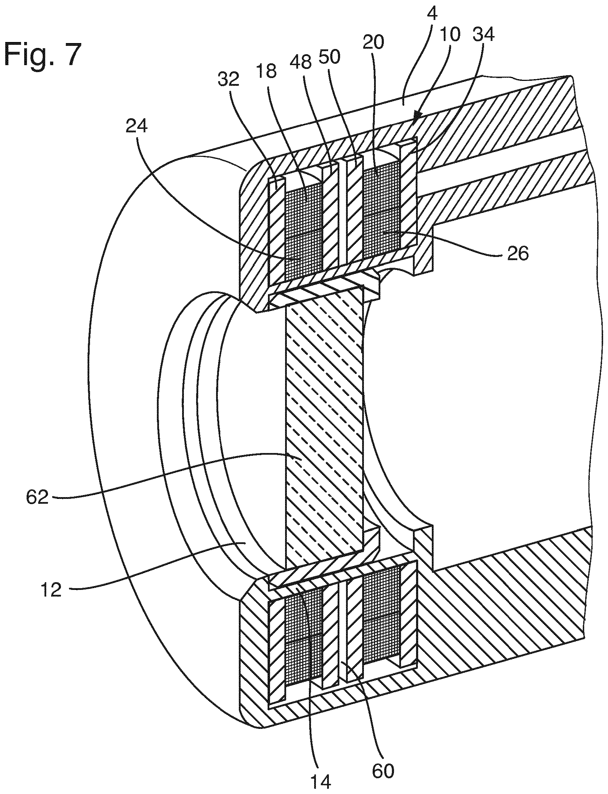

Figur 7 zeigt den aus Figur 6 bekannten und in den Endoskopschaft 4 eingebauten elektromagnetischen Aktuator 10 in einer schematisch vereinfachten perspektivischen Schnittansicht.

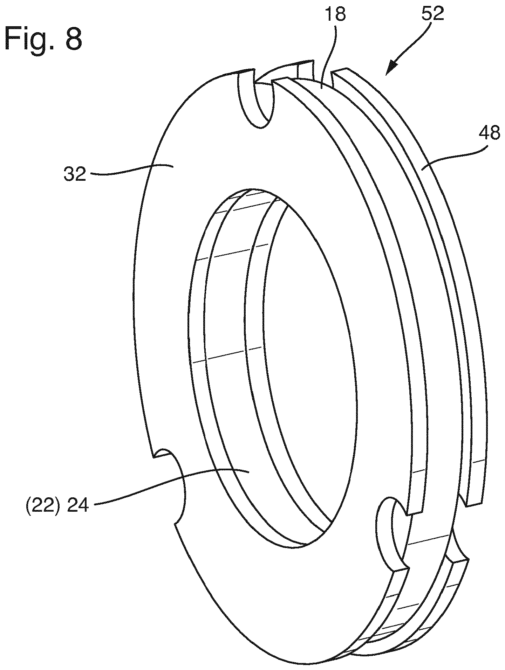

Figur 8 zeigt in vereinfachter schematischer und perspektivischer Darstellung eine vorgefertigte Baugruppe 52, 54, lediglich beispielhaft eine proximale vorgefertigte Baugruppe 54.

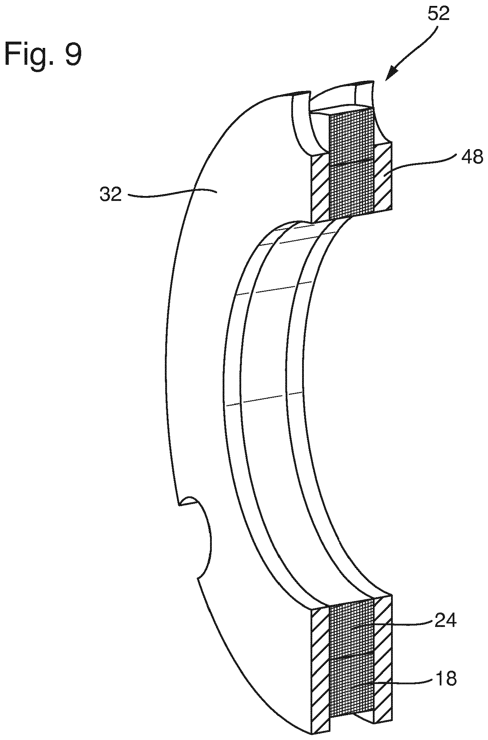

Figur 9 zeigt diese Baugruppe 54 in geschnittener schematisch vereinfachter perspektivischer Darstellung.

In den dargestellten Ausführungsbeispielen der Figuren 6 bis 9 sind die Permanentmagneten 18, 20 lediglich beispielhaft Ringmagnete. Sie umschließen die Spulen 22 vollständig. Beispielsweise werden die Permanentmagneten 18, 20 hergestellt, indem hartmagnetische Partikel in eine Kunststoffmatrix eingebettet werden. Hierzu ist insbesondere ein Spritzgussverfahren geeignet. Vorteilhaft ist es auf diese Weise möglich, die Bauteile der Baugruppen 52, 54 miteinander zu verbinden und gleichzeitig den entsprechenden Permanentmagneten 18, 20 der Baugruppe 52, 54 herzustellen.

Zur Kontaktierung der innenliegenden Spulen 22 ist in diesem Zusammenhang gemäß einem weiteren Ausführungsbeispiel vorgesehen, dass zumindest einer der Anschlussdrähte der innenliegenden Spule 22 durch den auf Basis einer Kunststoffmatrix hergestellten Permanentmagneten 18, 20 geführt wird.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezuqszeichenliste

- 2: Endoskop
- 4: Endoskopschaft
- 6: distales Ende
- 8: Handgriff
- 10: elektromagnetischer Aktuator
- 12: Läufer
- 14: Rohr
- 16: Stator
- 18, 18': distaler Permanentmagnet
- 20, 20': proximaler Permanentmagnet
- 22: Spule
- 24: distale Spule
- 26: proximale Spule
- 28: Außenseite der Spule
- 30: Außenseite des Stators
- 32: distaler Statorpolschuh
- 34: proximaler Statorpolschuh
- 36: zentraler Statorpolschuh
- 38: Innenseite der Spule
- 40: Außenseite der Permanentmagneten
- 42: erstes statisches Magnetfeld
- 44: zweites statisches Magnetfeld
- 46: elektromagnetisches Feld
- 48: distaler zentraler Stator-Teil-Polschuh
- 50: proximaler zentraler Stator-Teil-Polschuh
- 52: distale Baugruppe
- 54: proximale Baugruppe
- 56: distaler Anschlag
- 58: proximaler Anschlag
- 60: Luftspalt
- 62: Linse

- L: Längsaxialrichtung
- M: Mittenlängsachse
- R: Radialrichtung

## Patentansprüche

1. Elektromagnetischer Aktuator (10) für ein chirurgisches Instrument, umfassend: ein Rohr (14); einen außerhalb des Rohrs (14) angeordneten Stator (16) und einen im Rohr (14) gelagerten Läufer (12), der in einer Längsaxialrichtung (L) des Rohrs (14) in dem Rohr verschiebbar gelagert ist, wobei der Läufer (12) wenigstens teilweise ein paramagnetisches und/oder ferromagnetisches Material umfasst und durch Beaufschlagung mit einem elektromagnetischen Feld (46) in der Längsaxialrichtung (L) verschiebbar ist, wobei der Stator (16) einen distalen Permanentmagneten (18) und einen proximalen Permanentmagneten (20) umfasst, die in Längsaxialrichtung (L) gegensinnig gepolt sind, und zumindest eine elektrische Spule (22) zum Erzeugen des elektromagnetischen Felds (46) umfasst, **dadurch gekennzeichnet, dass** die Permanentmagnete (18, 20) auf einer dem Rohr (14) abgewandten Außenseite (28) der Spule (22) angeordnet sind.

2. Elektromagnetischer Aktuator nach Anspruch 1, wobei der distale und der proximale Permanentmagnet (18, 20) ein magnetisches Rückschlusselement für das von der elektrischen Spule (22) erzeugte Magnetfeld bilden.

3. Elektromagnetischer Aktuator (10) nach Anspruch 1 oder 2, wobei eine dem Rohr (14) abgewandte Außenseite (40) der Permanentmagnete (18, 20) zumindest eine Teilfläche einer dem Rohr (14) abgewandten Außenseite (30) des Stators (16) bildet.

4. Elektromagnetischer Aktuator (10) nach einem der Ansprüche 1 bis 3, wobei ein distales Ende des Stators (16) von einem distalen Statorpolschuh (32) und ein in Längsaxialrichtung (L) gegenüberliegendes proximales Ende von einem proximalen Statorpolschuh (34) gebildet sind, wobei insbesondere der Stator (16) einen zentralen Statorpolschuh (36) umfasst, der in Längsaxialrichtung (L) zwischen den Permanentmagneten (18, 20) angeordnet ist.

5. Elektromagnetischer Aktuator (10) nach Anspruch 4, wobei dass sich der zentrale Statorpolschuh (36) in einer zu der Längsaxialrichtung (L) senkrechten Radialrichtung (R) von der dem Rohr (14) zugewandten Innenseite (38) der Spule (22) bis zu der dem Rohr (14) abgewandten Außenseite (40) der Permanentmagneten (18, 20) erstreckt und die Spule (22) eine distale Spule (24) und eine proximale Spule (26) umfasst, wobei sich die beiden Spulen (24, 26) in Längsaxialrichtung (L) zu beiden Seiten des zentralen Statorpolschuhs (36) erstrecken und elektrisch so miteinander verbunden sind, dass die distale Spule (24) ein erstes Magnetfeld erzeugt, welches gleich zu einem von der proximalen Spule (26) erzeugten zweiten Magnetfeld orientiert ist, wobei insbesondere der zentrale Statorpolschuh (36) aus einem distalen zentralen Stator-Teil-Polschuh (48) und aus einem proximalen zentralen Stator-Teil-Polschuh (50) gebildet ist.

6. Elektromagnetischer Aktuator (10) nach Anspruch 5, wobei der distale Statorpolschuh (32), die distale Spule (24), der distale Permanentmagnet (18) und der distale zentrale Stator-Teil-Polschuh (48) eine vorgefertigte distale Baugruppe (52) bilden und der proximale zentrale Stator-Teil-Polschuh (50), die proximale Spule (26), der proximale Permanentmagnet (20) und der proximale Statorpolschuh (34) eine vorgefertigte proximale Baugruppe (54) bilden, wobei insbesondere die Bauteile der distalen und/oder der proximalen Baugruppe (52, 54) miteinander verklebt sind.

7. Elektromagnetischer Aktuator (10) nach einem der Ansprüche 4 bis 6, wobei sich zumindest einer der Statorpolschuhe (32, 34, 36) in einer zu der Längsaxialrichtung (L) senkrechten Radialrichtung (R) von einer dem Rohr (14) zugewandten Innenseite (38) der Spule (22) bis zu einer dem Rohr (14) abgewandten Außenseite (40) der Permanentmagneten (18, 20) erstrecken.

8. Elektromagnetischer Aktuator (10) nach einem der Ansprüche 1 bis 7, wobei die Permanentmagneten (18, 20) Ringmagnete sind, die die Spulen (22) umschließen oder der distale und der proximale Permanentmagnet (18, 20) jeweils aus zumindest einem Stabmagnet gebildet ist, wobei insbesondere der distale und der proximale Permanentmagnet (18, 20) jeweils aus mehreren Stabmagneten gebildet ist, wobei die den distalen und/oder den proximalen Permanentmagneten (18, 20) bildenden Stabmagneten gleichmäßig beabstandet entlang des Umfangs des Rohrs (14) angeordnet sind.

9. Elektromagnetischer Aktuator (10) nach einem der Ansprüche 1 bis 8, wobei zumindest einer der Permanentmagnete (18, 20) hartmagnetische Partikel umfasst, die in einer Kunststoffmatrix eingebettet sind, wobei dieser Permanentmagnet (18, 20) insbesondere in einem Spritzgussverfahren hergestellt ist, wobei insbesondere in zumindest einem Permanentmagneten (18, 20) zumindest ein Spulendraht der Spule (22) eingegossen ist.

10. Chirurgisches Instrument, insbesondere Endoskop (2), mit einem elektromagnetischen Aktuator (10) nach einem der Ansprüche 1 bis 9.

11. Verfahren zum Herstellen eines elektromagnetischen Aktuators (10) für ein chirurgisches Instrument, umfassend: ein Rohr (14); einen außerhalb des Rohrs (14) angeordneten Stator (16) und einen im Rohr (14) gelagerten Läufer (12), der in einer Längsaxialrichtung (L) des Rohrs (14) in dem Rohr (14) verschiebbar gelagert ist, wobei der Läufer (12) wenigstens teilweise ein paramagnetisches und/oder ferromagnetisches Material umfasst und durch Beaufschlagung mit einem elektromagnetischen Feld (46) in der Längsaxialrichtung (L) verschiebbar ist, wobei der Stator (16) einen distalen Permanentmagneten (18) und einen proximalen Permanentmagneten (20) umfasst, die in Längsaxialrichtung (L) gegensinnig gepolt sind, und zumindest eine elektrische Spule (22) zum Erzeugen des elektromagnetischen Felds (46) umfasst, **dadurch gekennzeichnet, dass** die Permanentmagneten (18, 20) auf einer dem Rohr (14) abgewandten Außenseite (28) der Spule (22) angeordnet werden.

12. Verfahren nach Anspruch 11, wobei die Permanentmagneten (18, 20) derart angeordnet werden, so dass eine dem Rohr (14) abgewandte Außenseite (40) der Permanentmagneten (18, 20) zumindest eine Teilfläche einer dem Rohr (14) abgewandten Außenseite (30) des Stators (16) bilden.

13. Verfahren nach Anspruch 11 oder 12, wobei ein distaler Statorpolschuh (32) distal des distalen Permanentmagneten (18) angeordnet wird und ein distales Ende des Stators (16) bildet und ein proximaler Statorpolschuh (34) proximal des proximalen Permanentmagneten (20) angeordnet wird und ein in Längsaxialrichtung (L) gegenüberliegendes proximales Ende bildet, wobei insbesondere ein zentraler Statorpolschuh (36) in Längsaxialrichtung (L) zwischen den Permanentmagneten (18, 20) angeordnet wird.

14. Verfahren nach einem der Ansprüche 11 bis 13, wobei sich der zentrale Statorpolschuh (36) in einer zu der Längsaxialrichtung (L) senkrechten Radialrichtung (R) von der dem Rohr (14) zugewandten Innenseite (38) der Spule (22) bis zu der dem Rohr (14) abgewandten Außenseite (40) der Permanentmagneten (18, 20) erstreckt und die Spule (22) eine distale Spule (24) und eine proximale Spule (26) umfasst, wobei der distale Statorpolschuh (32), die distale Spule (24), der distale Permanentmagnet (18) und der distale zentrale Stator-Teil-Polschuh (48) zu einer vorgefertigten distalen Baugruppe (52) und der proximale zentrale Stator-Teil-Polschuh (50), die proximale Spule (26), der proximale Permanentmagnet (20) und der proximale Statorpolschuh (34) zu einer vorgefertigten proximalen Baugruppe (54) zusammengefügt werden, wobei insbesondere die Bauteile der distalen und/oder der proximalen Baugruppe (52, 54) miteinander verklebt werden, und anschließend die vorgefertigte proximale Baugruppe (54) und die vorgefertigte distale Baugruppe (52) montiert werden, wobei die beiden Spulen (24, 26) elektrisch so miteinander verbunden werden, dass die distale Spule (24) ein erstes Magnetfeld erzeugt, welches gleich zu einem von der proximalen Spule (26) erzeugten zweiten Magnetfeld orientiert ist und der zentrale Statorpolschuh (36) aus dem proximalen zentralen Stator-Teil-Polschuh (50) und aus dem distalen zentralen Stator-Teil-Polschuh (48) gebildet ist.

15. Verfahren nach Anspruch 14, wobei die erste und/oder die zweite Baugruppe (52, 54) mit einem Permanentmagneten (18, 20) hergestellt wird, der hartmagnetische Partikel umfasst, die in einer Kunststoffmatrix eingebettet sind, wobei dieser Permanentmagnet (18, 20) in einem Spritzgussverfahren hergestellt wird und die Kunststoffmatrix gleichzeitig als Klebstoff für die Bauteile der ersten und/oder zweiten Baugruppe (52, 54) dient, wobei insbesondere in zumindest einem Permanentmagneten (18, 20) zumindest ein Spulendraht der Spule (22) eingegossen wird.

## Claims

1. An electromagnetic actuator (10) for a surgical instrument, comprising: a tube (14); a stator (16) arranged outside the tube (14) and a rotor (12) mounted in the tube (14) such that it can move in the tube in a longitudinal axial direction (L) of the tube (14), wherein the rotor (12) at least partially comprises a paramagnetic and/or ferromagnetic material and can be moved in the longitudinal axial direction (L) by application of an electromagnetic field (46), wherein the stator (16) comprises a distal permanent magnet (18) and a proximal permanent magnet (20) that are oppositely polarized in the longitudinal axial direction (L), and at least one electric coil (22) for generating the electromagnetic field (46), **characterized in that** the permanent magnets (18, 20) are arranged on an outer side (28) of the coil (22), facing away from the tube (14).

2. The electromagnetic actuator according to Claim 1, wherein the distal and the proximal permanent magnet (18, 20) form a magnetic return element for the magnetic field generated by the electric coil (22).

3. The electromagnetic actuator (10) according to Claim 1 or 2, wherein an outer side (40) of the permanent magnets (18, 20) facing away from the tube (14) forms at least one partial surface of an outer side (30) of the stator (16) facing away from the tube (14).

4. The electromagnetic actuator (10) according to any one of Claims 1 to 3, wherein a distal end of the stator (16) is formed by a distal stator pole shoe (32) and an opposite proximal end in the longitudinal axial direction (L) is formed by a proximal stator pole shoe (34), wherein the stator (16) comprises in particular a central stator pole shoe (36) which is arranged between the permanent magnets (18, 20) in the longitudinal axial direction (L).

5. The electromagnetic actuator (10) according to Claim 4, wherein the central stator pole shoe (36) extends in a radial direction (R) perpendicular to the longitudinal axial direction (L) from the inner side (38) of the coil (22) facing the tube (14) up to the outer side (40) of the permanent magnets (18, 20) facing away from the tube (14) and the coil (22) comprises a distal coil (24) and a proximal coil (26), wherein the two coils (24, 26) extend in the longitudinal axial direction (L) on both sides of the central stator pole shoe (36) and are electrically connected to one another such that the distal coil (24) generates a first magnetic field which is oriented identically to a second magnetic field generated by the proximal coil (26), wherein the central stator pole shoe (36) is in particular formed from a distal central stator part pole shoe (48) and from a proximal central stator part pole shoe (50).

6. The electromagnetic actuator (10) according to Claim 5, wherein the distal stator pole shoe (32), the distal coil (24), the distal permanent magnet (18) and the distal central stator part pole shoe (48) form a prefabricated distal assembly (52) and the proximal central stator part pole shoe (50), the proximal coil (26), the proximal permanent magnet (20) and the proximal stator pole shoe (34) form a prefabricated proximal assembly (54), wherein the components of the distal and/or the proximal assembly (52, 54) are in particular bonded to one another.

7. The electromagnetic actuator (10) according to any one of Claims 4 to 6, wherein at least one of the stator pole shoes (32, 34, 36) extend in a radial direction (R) perpendicular to the longitudinal axial direction (L) from an inner side (38) of the coil (22) facing the tube (14) up to an outer side (40) of the permanent magnets (18, 20) facing away from the tube (14).

8. The electromagnetic actuator (10) according to any one of Claims 1 to 7, wherein the permanent magnets (18, 20) are annular magnets which enclose the coils (22) or the distal and the proximal permanent magnet (18, 20) are each formed from at least one bar magnet, wherein the distal and the proximal permanent magnet (18, 20) are each formed from multiple bar magnets, wherein the bar magnets forming the distal and/or the proximal permanent magnets (18, 20) are arranged uniformly spaced along the circumference of the tube (14).

9. The electromagnetic actuator (10) according to any one of Claims 1 to 8, wherein at least one of the permanent magnets (18, 20) comprises magnetically hard particles which are embedded in a plastic matrix, wherein said permanent magnet (18, 20) is in particular produced using an injection molding method, wherein at least one coil wire of the coil (22) is in particular molded in at least one permanent magnet (18, 20).

10. A surgical instrument, in particular an endoscope (2), having an electromagnetic actuator (10) according to any one of Claims 1 to 9.

11. A method for producing an electromagnetic actuator (10) for a surgical instrument, comprising: a tube (14); a stator (16) arranged outside the tube (14) and a rotor (12) mounted in the tube (14) such that it can move in the tube (14) in a longitudinal axial direction (L) of the tube (14), wherein the rotor (12) at least partially comprises a paramagnetic and/or ferromagnetic material and can be moved in the longitudinal axial direction (L) by application of an electromagnetic field (46), wherein the stator (16) comprises a distal permanent magnet (18) and a proximal permanent magnet (20) that are oppositely polarized in the longitudinal axial direction (L), and at least one electric coil (22) for generating the electromagnetic field (46), **characterized in that** the permanent magnets (18, 20) are arranged on an outer side (28) of the coil (22), facing away from the tube (14).

12. The method according to Claim 11, wherein the permanent magnets (18, 20) are arranged in such a manner that an outer side (40) of the permanent magnets (18, 20) facing away from the tube (14) forms at least one partial surface of an outer side (30) of the stator (16) facing away from the tube (14).

13. The method according to Claim 11 or 12, wherein a distal stator pole shoe (32) is arranged distally to the distal permanent magnet (18) and forms a distal end of the stator (16) and a proximal stator pole shoe (34) is arranged proximally to the proximal permanent magnet (20) and forms an opposite proximal end in the longitudinal axial direction (L), wherein a central stator pole shoe (36) is in particular arranged between the permanent magnets (18, 20) in the longitudinal axial direction (L).

14. The method according to any one of Claims 11 to 13, wherein the central stator pole shoe (36) extends in a radial direction (R) perpendicular to the longitudinal axial direction (L) from the inner side (38) of the coil (22) facing the tube (14) up to the outer side (40) of the permanent magnets (18, 20) facing away from the tube (14) and the coil (22) comprises a distal coil (24) and a proximal coil (26), wherein the distal stator pole shoe (32), the distal coil (24), the distal permanent magnet (18) and the distal central stator part pole shoe (48) are joined together to form a prefabricated distal assembly (52) and the proximal central stator part pole shoe (50), the proximal coil (26), the proximal permanent magnet (20) and the proximal stator pole shoe (34) are joined together to form a prefabricated proximal assembly (54), wherein the components of the distal and/or the proximal assembly (52, 54) are in particular bonded to one another, and the prefabricated proximal assembly (54) and the prefabricated distal assembly (52) are subsequently mounted, wherein the two coils (24, 26) are electrically connected to one another such that the distal coil (24) generates a first magnetic field which is oriented identically to a second magnetic field generated by the proximal coil (26) and the central stator pole shoe (36) is formed from the proximal central stator part pole shoe (50) and from the distal central stator part pole shoe (48).

15. The method according to Claim 14, wherein the first and/or the second assembly (52, 54) is/are produced with a permanent magnet (18, 20) which comprises magnetically hard particles which are embedded in a plastic matrix, wherein said permanent magnet (18, 20) is produced using an injection molding method and the plastic matrix simultaneously serves as an adhesive for the components of the first and/or second assembly (52, 54), wherein at least one coil wire of the coil (22) is molded in particular in at least one permanent magnet (18, 20).

## Revendications

1. Dispositif d'actionnement électromagnétique (10) pour un instrument chirurgical, comprenant : un tube (14) ; un stator (16) disposé à l'extérieur du tube (14) et un rotor (12) monté dans le tube (14), le rotor (12) étant monté coulissant dans le tube (14) dans une direction axiale longitudinale (L) du tube (14), le rotor (12) comprenant au moins en partie un matériau paramagnétique et/ou ferromagnétique et étant apte à coulisser dans la direction axiale longitudinale (L) par exposition à un champ électromagnétique (46), le stator (16) comprenant un aimant permanent distal (18) et un aimant permanent proximal (20) qui sont polarisés dans des directions opposées dans la direction axiale longitudinale (L), et comprenant au moins une bobine électrique (22) pour générer le champ électromagnétique (46), **caractérisé en ce que** les aimants permanents (13, 20) sont agencés sur un côté extérieur (28) de la bobine (22) qui est opposé au tube (14).

2. Dispositif d'actionnement électromagnétique selon la revendication 1, dans lequel l'aimant permanent distal et l'aimant permanent proximal (18, 20) forment un élément de reflux magnétique pour le champ magnétique généré par la bobine électrique (22).

3. Dispositif d'actionnement électromagnétique (10) selon la revendication 1 ou la revendication 2, dans lequel un côté extérieur (40) des aimants permanents (13, 20) opposé au tube (14) forme au moins une surface partielle d'une surface extérieure (30) du stator (16) opposée au tube (14).

4. Dispositif d'actionnement électromagnétique (10) selon l'une quelconque des revendications 1 à 3, dans lequel une extrémité distale du stator (16) est formée par une pièce polaire distale (32) de stator et une extrémité proximale opposée dans la direction axiale longitudinale (L) est formée par une pièce polaire proximale (34) de stator, le stator (16) comprenant en particulier une pièce polaire centrale (36) de stator disposée dans la direction axiale longitudinale (L) entre les aimants permanents (18, 20).

5. Dispositif d'actionnement électromagnétique (10) selon la revendication 4, dans lequel la pièce polaire centrale (36) de stator s'étend dans une direction radiale (R) perpendiculaire à la direction axiale longitudinale (L) du tube (14), depuis le côté intérieur (38) de la bobine (22) tourné vers le tube (14) jusqu'au côté extérieur (40) des aimants permanents (18), 20), et la bobine (22) comprend une bobine distale (24) et une bobine proximale (26), les deux bobines (24, 26) s'étendant dans la direction axiale longitudinale (L) de part et d'autre de la pièce polaire centrale (36) de stator et étant reliées électriquement entre elles de telle sorte que la bobine distale (24) génère un premier champ magnétique qui est orienté de manière identique à un deuxième champ magnétique généré par la bobine proximale (26), la pièce polaire centrale (36) de stator étant en particulier formée d'une pièce polaire de partie centrale distale (48) de stator et d'une pièce polaire de partie centrale proximale (50) de stator.

6. Dispositif d'actionnement électromagnétique (10) selon la revendication 5, dans lequel la pièce polaire distale (32) de stator, la bobine distale (24), l'aimant permanent distal (18) et la pièce polaire de partie centrale distale (48) de stator forment un ensemble distal préfabriqué (52), et la pièce polaire de partie centrale proximale (50) de stator, la bobine proximale (26), l'aimant permanent proximal (20) et la pièce polaire proximale (34) de stator forment un ensemble proximal préfabriqué (54), les composants de l'ensemble distal et/ou proximal (52, 54) étant notamment collés ensemble.

7. Dispositif d'actionnement électromagnétique (10) selon l'une quelconque des revendications 4 à 6, dans lequel au moins l'une des pièces polaires (32, 34, 36) de stator s'étend dans une direction radiale (R) perpendiculaire à la direction axiale longitudinale (L) depuis un côté intérieur (38) de la bobine (22) tourné vers le tube (14) jusqu'à un côté extérieur (40) des aimants permanents (18, 20) opposé au tube (14).

8. Dispositif d'actionnement électromagnétique (10) selon l'une quelconque des revendications 1 à 7, dans lequel les aimants permanents (18, 20) sont des aimants annulaires entourant les bobines (22), ou l'aimant permanent distal et l'aimant permanent proximal (18, 20) sont chacun formés d'au moins un aimant en barreau, l'aimant permanent distal et l'aimant permanent proximal (18, 20) étant en particulier chacun formés d'une pluralité d'aimants en barreaux, les aimants en barreaux formant l'aimant permanent distal et/ou l'aimant permanent proximal (18, 20) étant agencés de façon régulièrement espacée le long de la circonférence du tube (14).

9. Dispositif d'actionnement électromagnétique (10) selon l'une quelconque des revendications 1 à 8, dans lequel au moins un des aimants permanents (18, 20) comprend des particules magnétiques dures enrobées dans une matrice en matière plastique, ledit aimant permanent (18, 20) étant en particulier fabriqué par un procédé de moulage par injection, au moins un fil de bobine de la bobine (22) étant en particulier noyé dans au moins un aimant permanent (18, 20).

10. Instrument chirurgical, en particulier un endoscope (2), comprenant un dispositif d'actionnement électromagnétique (10) selon l'une quelconque des revendications 1 à 9.

11. Procédé de fabrication d'un dispositif d'actionnement électromagnétique (10) pour un instrument chirurgical comprenant : un tube (14) ; un stator (16) disposé à l'extérieur du tube (14) ; et un rotor (12) supporté à l'intérieur du tube (14) et étant apte à coulisser dans le tube (14) selon une direction axiale longitudinale (L) du tube (14), le rotor (12) comprenant au moins en partie un matériau paramagnétique et/ou ferromagnétique et étant apte à coulisser dans la direction axiale longitudinale (L) par exposition à un champ électromagnétique (46), le stator (16) comprenant un aimant permanent distal (18) et un aimant permanent proximal (20) qui sont polarisés dans des directions opposées selon la direction axiale longitudinale (L), et comprenant au moins une bobine électrique (22) pour générer le champ électromagnétique (46), **caractérisé en ce que** les aimants permanents (13, 20) sont agencés sur un côté extérieur (28) de la bobine (22) qui est opposé au tube (14).

12. Procédé selon la revendication 11, dans lequel les aimants permanents (18, 20) sont disposés de telle sorte qu'un côté extérieur (40) des aimants permanents (18, 20) opposé au tube (14) forme au moins une surface partielle d'un côté extérieur (30) du stator (16) opposé au tube (14).

13. Procédé selon la revendication 11 ou la revendication 12, dans lequel une pièce polaire distale (32) de stator est agencée de manière distale par rapport à l'aimant permanent distal (18) et forme une extrémité distale du stator (16), et une pièce polaire proximale (34) de stator est agencée de manière proximale par rapport à l'aimant permanent proximal (20) et forme une extrémité proximale opposée dans la direction axiale longitudinale (L), une pièce polaire centrale (36) de stator étant en particulier agencée dans la direction axiale longitudinale (L) entre les aimants permanents (13, 20).

14. Procédé selon l'une quelconque des revendications 11 à 13, dans lequel la pièce polaire centrale (36) de stator s'étend dans une direction radiale (R) perpendiculaire à la direction axiale longitudinale (L) du tube (14), depuis le côté intérieur (38) de la bobine (22) tourné vers le tube (14) jusqu'au côté extérieur (40) des aimants permanents (18), 20), et la bobine (22) comprend une bobine distale (24) et une bobine proximale (26), la pièce polaire distale (32) de stator, la bobine distale (24), l'aimant permanent distal (18) et la pièce polaire de partie centrale distale (48) de stator forment un ensemble distal préfabriqué (52), et la pièce polaire de partie centrale proximale (50) de stator, la bobine proximale (26), l'aimant permanent proximal (20) et la pièce polaire proximale (34) de stator sont assemblés pour former un ensemble proximal préfabriqué (54), les composants de l'ensemble distal et/ou proximal (52, 54) étant en particulier collés ensemble, et ensuite l'ensemble proximal préfabriqué (54) et l'ensemble distal préfabriqué (52) sont assemblés, les deux bobines (24, 26) étant électriquement connectées l'une à l'autre de telle sorte que la bobine distale (24) génère un premier champ magnétique qui est orienté de la même manière qu'un deuxième champ magnétique généré par la bobine proximale (26), et la pièce polaire centrale (36) de stator est formée depuis la pièce polaire de partie centrale proximale (50) de stator et de la pièce polaire de partie centrale distale (48) de stator.

15. Procédé selon la revendication 14, dans lequel le premier et/ou le deuxième ensemble (52, 54) est fabriqué avec un aimant permanent (18, 20) comprenant des particules magnétiques dures enrobées dans une matrice en matière plastique, cet aimant permanent (18, 20) étant fabriqué par un procédé de moulage par injection et la matrice en matière plastique servant simultanément d'adhésif pour les composants du premier et/ou du deuxième ensemble (52, 54), au moins un fil de bobine de la bobine (22) étant en particulier noyé dans au moins un aimant permanent (18, 20).
